# EUROPEAN PATENT APPLICATION

(11) **EP 1 178 030 A2**
(43) Date of publication of application: **06.02.2002**
(21) Application number: 01118203.7
(22) Date of filing: 28.07.2001
(51) Int. Cl.: C07C 41/01, C07C 41/40, C07C 41/42, C07C 43/13

(54) **Process for recovering ditrimethylolpropane**

(30) Priority: 03.08.2000 JP 2000235578; 03.08.2000 JP 2000235580; 03.08.2000 JP 2000235582; 03.08.2000 JP 2000235583; 03.08.2000 JP 2000235584
(71) Applicant: MITSUBISHI GAS CHEMICAL COMPANY, INC., Chiyoda-ku, Tokyo (JP)
(72) Inventor: Ninomiya, Teruyuki, Kurashiki-shi, Okayama (JP); Watanabe, Toshio, Kurashiki-shi, Okayama (JP); Iwamoto, Atsushi, Kurashiki-shi, Okayama (JP); Miyashita, Soemu, Kurashiki-shi, Okayama (JP); Watanabe, Masafumi, Kurashiki-shi, Okayama (JP)
(74) Representative: Gille Hrabal Struck Neidlein Prop Roos

(57) **Abstract**

The present invention provides processes for recovering ditrimethylolpropane from a still residue obtained by extracting and then distilling off trimethylolpropane from a reaction solution obtained by reacting n-butyraldehyde with formaldehyde in the presence of a basic catalyst.
One process comprises acid decomposition of formals in the still residue.
A second process comprises removal from the still residue of components having a higher boiling point than that of ditrimethylolpropane followed by crystallization of the resultant product.

## Description

### BACKGROUND OF THE INVENTION

### Field of the Invention

The present invention relates to a process for recovering ditrimethylolpropane (hereinafter referred to merely as "di-TMP") by-produced upon the production of trimethylolpropane (hereinafter referred to merely as "TMP"). The thus recovered di-TMP is useful as a raw material for polyacrylate resins, polyether polyol resins, polyurethane resins, alkyd resins, synthetic lubricating oils or the like.

### Description of the Prior Art

TMP has been industrially produced by subjecting n-butyraldehyde (hereinafter referred to merely as "NBD") and formaldehyde to aldol condensation and crossed Cannizzaro reaction in the presence of a basic catalyst (refer to U.S. Patent No. 3,097,245, etc.). Also, di-TMP is by-produced upon the production of TMP, and recovered from a reaction mixture obtained thereupon.

More specifically, a reaction solution obtained by the reaction between NBD and formaldehyde which may be concentrated or non-concentrated, is extracted with a solvent to obtain a TMP extract (crude TMP) containing substantially no sodium formate. When the TMP extract is purified by distillation under a high vacuum, the resultant still residue contains 1 to 10% of TMP and 20 to 50% of di-TMP.

Japanese Patent Application Laid-open No. Showa 47(1972)-30611 discloses the method of obtaining purified di-TMP from the still residue by crystallization using ethyl acetate. However, in this method, since an organic solvents such as ethyl acetate is used when the di-TMP is obtained by subjecting the still residue of the crude TMP to recrystallization, it is not possible to obtain high-purity di-TMP. This is because bis-TMP cannot be removed from di-TMP by the crystallization using such an organic solvent.

Also, Japanese Patent Application Laid-open No. Showa 49 (1974) -133311 discloses the method of subjecting the still residue of the crude TMP to crystallization using an aqueous solvent in the presence of sodium formate. However, in this method using water as the solvent, if the still residue is tinted, it is substantially impossible to remove coloring components therefrom.

### SUMMARY OF THE INVENTION

An object of the present invention is to provide a process for obtaining a high-purity di-TMP containing no coloring components when separating and recovering di-TMP from the still residue of crude TMP obtained upon the production of TMP.

As a result of extensive researches in view of the above object, the inventors have found that 1) a high-purity di-TMP is obtained by adding an acid to still residue of crude TMP to thereby decompose a straight-chain formal compound produced by the reaction between 2 molecules of TMP and formaldehyde contained in the still residue (hereinafter referred to merely as "bis-TMP") which cannot be removed from the bis-TMP by crystallization; 2) it is effective to add a scavenger for removing decomposed products such as alcohols and hydroxylamine salts because by-products are formed by the acid decomposition; 3) untinted higher-purity di-TMP is obtained by using film evaporator or crystallization using a solvent in combination with the acid decomposition since the coloring components have a higher boiling point than that of di-TMP (these items 1) through 3) are related to the first invention); and 4) the coloring components are removed by first removing higher boiling components from the still residue and then subjecting the resultant product to crystallization (the item 4) is related to the second invention). The present invention has been accomplished on the basis of these finding.

Thus, the present invention provides the following aspects:
(1) a process for recovering ditrimethylolpropane from a still residue obtained by extracting and then distilling off trimethylolpropane from a reaction solution obtained by reacting n-butyraldehyde with formaldehyde in the presence of a basic catalyst, said process comprising: subjecting a formal compound contained in the still residue to acid decomposition;
(2) a process according to the above aspect (1), wherein the acid decomposition of the formal compound is performed at a temperature of 20 to 180°C using a mineral acid and/or an organic acid;
(3) a process according to the above aspect (2), wherein at least one compound selected from the group consisting of alcohols and hydroxylamine salts is added to the still residue together with the acid;
(4) a process according to any one of the above aspects (1) to (3), comprising the steps of: i) subjecting the formal compound contained in the still residue to acid decomposition, ii) removing high-boiling components having a higher boiling point than that of ditrimethylolpropane, from the still residue, and iii) subjecting the resultant product to crystallization using a solvent;
(5) a process according to any one of the above aspects (1) to (3), wherein after the formal compound contained in a distillate obtained by removing the high-boiling components having a higher boiling point than that of ditrimethylolpropane, is subjected to acid decomposition, the resultant product is subjected to crystallization using a solvent;
(6) a process according to the above aspect (4) or (5), wherein the removal of the high-boiling components having a higher boiling point than that of ditrimethylolpropane, is performed by molecular distillation;
(7) a process according to any one of the above aspects (1) to (3), wherein after the still residue is first subjected to crystallization using a solvent and then the formal compound contained in the resultant crystallized product is subjected to acid decomposition, the resultant reaction mixture obtained from the acid decomposition is subjected to crystallization;
(8) a process for recovering ditrimethylolpropane from a still residue obtained by extracting and then distilling off trimethylolpropane from a reaction solution obtained by reacting n-butyraldehyde with formaldehyde in the presence of a basic catalyst, said process comprising: removing high-boiling components having a higher boiling point than that of ditrimethylolpropane, from the still residue, and subjecting the resultant product to crystallization;
(9) a process according to the above aspect (8), wherein the removal of the high-boiling components having a higher boiling point than that of ditrimethylolpropane, is performed by molecular distillation ; and
(10) a process according to the above aspect (8) or (9), wherein the solvent used upon the crystallization is water.

### DESCRIPTION OF THE PREFERRED EMBODIMENTS

The ditrimethylolpropane (di-TMP) of the present invention and bistrimethylolpropane (bis-TMP), are represented by the following formulae (I) and (II), respectively.

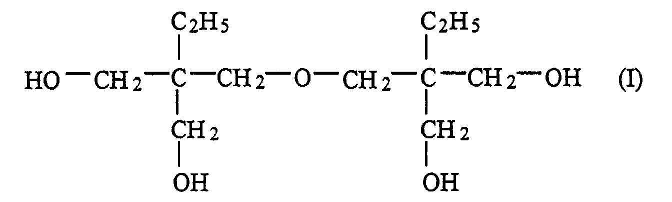

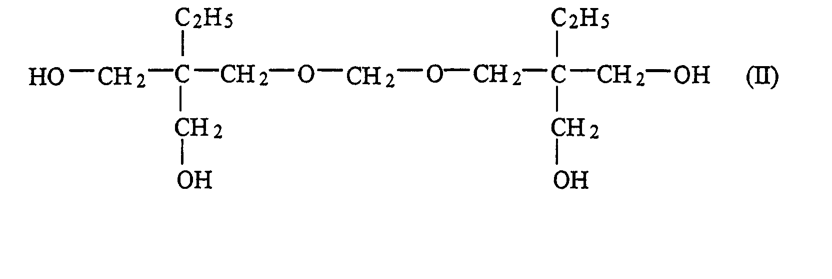

In the process of the present invention, when TMP is separated from a reaction solution obtained by the reaction between NBD and formaldehyde, the reaction solution in either concentrated or non-concentrated state is subjected to extraction with a solvent to obtain a TMP extract (crude TMP) containing substantially no sodium formate.

Examples of the solvent used upon the extraction of TMP include aliphatic esters such as butyl acetate and ethyl acetate; aliphatic ketones such as methylethyl ketone, methylisobutyl ketone and diisopropyl ketone; alcohols such as isobutanol, amyl alcohol, hexyl alcohol and cyclohexanol; aldehydes such as isobutyraldehyde and n-butyraldehyde; and mixed solvents thereof.

The crude TMP produced by extracting TMP from the reaction solution obtained by the reaction between NBD and formaldehyde, is purified by distillation under high vacuum to separate a TMP product from the still residue. The di-TMP is recovered from the still residue.

In the first invention, an acid and/or a hydroxylamine salt is added to the still residue to subject the bis-TMP to decomposition (hereinafter referred to as "acid decomposition reaction").

The acid decomposition reaction may be conducted using a mineral acid such as hydrochloric acid and sulfuric acid. In the case where high-boiling components or salts contained in the still residue have been previously removed by distillation or crystallization, the reaction may be conducted using phosphoric acid or an organic acid such as p-toluenesulfonic acid. In addition, the acid decomposition reaction may be performed using a hydroxylamine salt such as hydroxylamine sulfate and hydroxylamine hydrochloride. Ordinary industrial products of these acids and hydroxylamine salts may be directly used without any further purification

When the still residue is reacted with the acid such as sulfuric acid, the bis-TMP contained therein is decomposed. However, in this reaction, TMP-derived cyclic formal (hereinafter referred to merely as "CMF") obtained by the reaction between one TMP molecule and one formaldehyde molecule, and di-TMP-derived cyclic formal (hereinafter referred to merely as "CDF") obtained by the reaction between one di-TMP molecule and one formaldehyde molecule, are additionally by-produced.

The TMP-derived cyclic formal (CMF) and di-TMP-derived cyclic formal (CDF) are represented by the following formulae (III) and (IV), respectively.

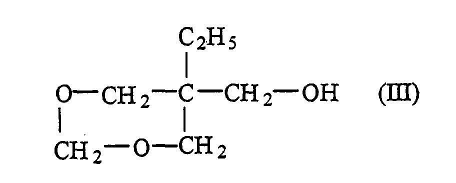

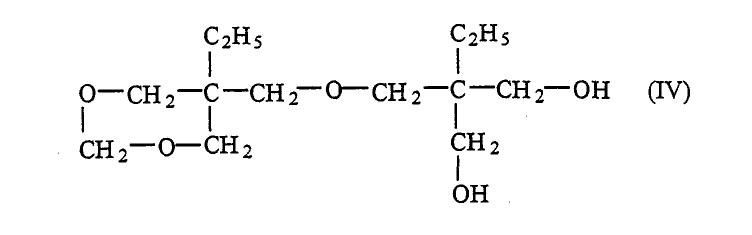

Similarly to bis-TMP, it is difficult to separate CDF from di-TMP by crystallization. Unlike bis-TMP, CDF is readily separable from di-TMP by distillation. However, since di-TMP is consumed when CDF is formed, the formation of CDF itself results in low recovery of di-TMP. Therefore, when the acid is added to the still residue for the acid decomposition thereof, it is preferred to further add a scavenger for removing decomposed products such as formaldehyde or derivatives thereof to inhibit the formation of CDF. Examples of the scavenger for removing formaldehyde or derivatives thereof are alcohols and hydroxylamine salts.

More specifically, in the first invention, it is preferred to add at least one compound selected from the group consisting of alcohols and hydroxylamine salts as a scavenger for decomposed products, together with the acid upon the acid decomposition reaction.

Examples of the alcohols used as the scavenger for decomposed products, include monohydric alcohols such as methyl alcohol and ethyl alcohol; and polyhydric alcohols such as ethylene glycol, propylene glycol and trimethylol propane. Examples of the hydroxylamine salts include hydroxylamine sulfate and hydroxylamine hydrochloride. Ordinary industrial products of these alcohols and hydroxylamine salts may be directly used without any further purification

The amount of the acid and/or the hydroxylamine salt used upon the acid decomposition reaction is 0.2 to 10% by weight, preferably 0.3 to 1.0% by weight based on the weight of the still residue. The scavenger for decomposed products is used in an amount of 1 to 30 moles per one mole of bis-TMP contained in the still residue.

The acid decomposition reaction is performed while stirring at a temperature of 20 to 180°C, preferably 70 to 110°C for 1 to 20 hours, preferably 3 to 6 hours.

Meanwhile, when the scavenger for decomposed products is used in the first invention, formaldehyde or derivatives thereof may be converted into dimethoxymethane or the like. It is preferred to rapidly discharge these products out of the reaction system. For this reason, the distillation may also be conducted in parallel during the acid decomposition reaction.

After completion of the acid decomposition reaction, the resultant reaction mixture was successively subjected to neutralization, removal of low-boiling components and removal of salts and high-boiling components having a higher boiling point than that of di-TMP to obtain a high-purity di-TMP.

As the neutralizer, there may be used aqueous solutions of sodium carbonate, sodium hydrogencarbonate, sodium hydroxide or the like.

The neutralization procedure may be conducted by adding the neutralizer little by little to the reaction mixture obtained by the acid decomposition reaction, and continued until the pH value thereof reaches 7.

The low-boiling components may be removed by distillation. Here, the "low-boiling components" mean those components having a lower boiling point than that of di-TMP, and mainly include CMF and TMP. These low-boiling components may be readily removed by distillation since the boiling points thereof are far lower than that of di-TMP. The distillation temperature is in the range of 140 to 200°C, preferably 150 to 180°C, and the distillation pressure is 670 Pa or lower, preferably 130 Pa or lower.

The removal of the salts and the high-boiling components having a higher boiling point than that of di-TMP may be effectively conducted by molecular distillation, specially film evaporator. The temperature used for the film evaporator is in the range of 130 to 280°C, preferably 160 to 250°C, and the pressure is 130 Pa or lower.

Also, in the first invention, the reaction mixture obtained by the acid decomposition reaction may be subjected to crystallization by adding a solvent thereto to obtain purified di-TMP.

Examples of the solvent used for the crystallization of di-TMP include organic solvents, e.g., aliphatic ethers such as dioxane and tetrahydrofuran; aliphatic esters such as ethyl acetate and butyl acetate; aliphatic ketones such as acetone and methylisobutyl ketone; and aliphatic alcohols such as hexyl alcohol and heptyl alcohol. These solvents may be used alone or in the form of a mixture of any two or more thereof.

The amount of the solvent used is 0.5 to 10 parts by weight, preferably 1 to 4 parts by weight based on one part by weight of the still residue obtained after the recovery of TMP. When the amount of the solvent used is less than 0.5 part by weight, the resultant crystals are deteriorated in quality, or no crystals are obtainable. When the amount of the solvent used is more than 10 parts by weight, the burden required for the recovery of the solvent becomes large, resulting in industrially disadvantageous process.

In the crystallization of di-TMP, the reaction mixture obtained by the acid decomposition reaction is mixed with the solvent under heating until a transparent solution is obtained. Then, the resultant solution is slowly cooled while stirring. In this case, the reaction mixture is usually heated to a temperature of 60 to 80°C, and then the resultant solution is cooled to about room temperature. In order to obtain the crystals at higher yield, the solution is cooled to a temperature lower than room temperature, preferably 0 to 1°C. The thus obtained crystals are washed, filtered, centrifugally separated and then dried to obtain a high-purity di-TMP.

Meanwhile, the crystal-washing solution obtained upon the crystallization of di-TMP and the solvent recovered upon the drying may be reused as the solvent for the crystallization.

In the first invention, when the acid and/or the hydroxylamine salt is added to the still residue obtained after distilling off TMP, bis-TMP may be selectively decomposed. Therefore, it becomes possible to readily obtain a high-purity di-TMP.

Further, in the first invention, the formation of CDF is avoided by adding the scavenger for decomposed products during the acid decomposition reaction, so that the di-TMP is produced at a high yield. Accordingly, in the first invention, even if the still residue obtained after distilling off TMP contains a considerable amount of bis-TMP, it is possible to obtain a high-quality di-TMP therefrom at a high yield.

Furthermore, since the process according to the first invention comprises the steps of i) subjecting the formal compound contained in the still residue to acid decomposition; ii) removing high-boiling components having a higher boiling point than that of ditrimethylolpropane, from the still residue; and iii) subjecting the resultant product to crystallization using a solvent, it is possible to effectively recover the di-TMP.

The present inventors have found that coloring components contained in the di-TMP products are compounds having a higher boiling point than that of di-TMP. Therefore, it is desirable to previously remove the high-boiling components from the still residue before the acid decomposition reaction. The removal of the high-boiling components from the still residue before the acid decomposition reaction may be suitably performed by either distillation or crystallization using a solvent. When the high-boiling components are removed from the still residue, substantially whole amounts of coloring components and salts such as sodium formate can be eliminated therefrom.

In the case where the high-boiling components are removed from the still residue by distillation, molecular distillation, specially the film evaporator is used to effectively distill off di-TMP. The temperature used for the film evaporator is in the range of 130 to 280°C, preferably 160 to 250°C, and the pressure used therefor is 130 Pa or lower. As the temperature for the film evaporator is lowered, the ratio of bis-TMP to di-TMP on the distillate side is reduced, so that the removal efficiency of the coloring components is improved.

When the high-boiling components are removed from the still residue by crystallization using a solvent, the crystallization may be performed in the substantially same manner as the above crystallization procedure conducted by adding the solvent to the reaction mixture obtained by the acid decomposition for obtaining the purified di-TMP. Therefore, the solvents usable in the crystallization for removing the high-boiling components from the still residue, may be usually the same as those used in the crystallization procedure for obtaining the purified di-TMP. Namely, when both the crystallization procedures are performed using the same solvent, the crystal washing solution obtained upon the crystallization of di-TMP and the solvent recovered upon the drying may be used as the solvent for the crystallization for removing the high-boiling components from the still residue.

Thus, when the still residue is subjected to the acid decomposition reaction after removing the high-boiling components therefrom, the bis-TMP is selectively decomposed into TMP and CMF. Therefore, when the reaction mixture is successively subjected to crystallization using a solvent, it is possible to readily obtain colorless high-purity di-TMP from the still residue even if the still residue contains a large amount of coloring components.

Namely, when the still residue is subjected to the acid decomposition reaction after removing the high-boiling components therefrom, it is possible to obtain a high purity di-TMP containing no coloring components even though the still residue obtained after distilling off TMP exhibits a Gardner color scale of about 18.

In the second invention, there is provided a process for obtaining di-TMP containing no coloring components by first removing the high-boiling components from the still residue and then subjecting the resultant product to crystallization.

The removal of the high-boiling components having a higher boiling point than that of ditrimethylolpropane from the still residue may be effectively performed by distilling off di-TMP from the still residue by a molecular distillation, specially film evaporator. The operating conditions of the film evaporator may be performed as same as described above.

Then, the resultant distillate is purified by crystallization method. The solvent used for the crystallization is preferably water because the use of water is most effective to separate di-TMP from bis-TMP. When the distillate obtained by the film evaporator contains almost no bis-TMP, the crystallization may also be conducted using an organic solvent, e.g., aliphatic ethers such as dioxane and tetrahydrofuran; aliphatic esters such as ethyl acetate and butyl acetate; aliphatic ketones such as acetone and methylisobutyl ketone; and aliphatic alcohols such as hexyl alcohol and heptyl alcohol If the distillate contains a large amount of TMP, the distillate may be further distilled to remove and recover the TMP therefrom. The amount of the solvent used for the crystallization is 0.5 to 10 parts by weight, preferably 1 to 4 parts by weight based on one part by weight of the distillate.

In the crystallization of the di-TMP, the distillate obtained by the film evaporator and the solvent are first mixed with each other under heating until a transparent solution is obtained, and then the resultant solution is slowly cooled while stirring. Usually, the mixture of the distillate and the solvent is heated to 60 to 80°C, and then the resultant solution is cooled to about room temperature. In order to obtain crystals at a much higher yield, the solution is cooled to a temperature lower than room temperature, preferably 0 to 1°C. The obtained crystals are washed, filtered, centrifugally separated and then dried to obtain a high-purity di-TMP.

Meanwhile, the crystal washing solution obtained upon the crystallization of di-TMP and the solvent recovered upon the drying may be reused for the crystallization.

In the second invention, since the still residue obtained after distilling off TMP is subjected to the film evaporator, it is possible to effectively remove the coloring components therefrom. In addition, when the distillate obtained by the film evaporator is further subjected to crystallization by adding a solvent thereto, it is possible to readily obtain a high-purity di-TMP.

Namely, even if the still residue obtained after distilling off TMP exhibits a Gardner color scale of about 13, when the still residue is subjected to the film evaporator, it is possible to obtain di-TMP containing no coloring components. Further, when the distillate obtained by the film evaporator is subjected to crystallization, the salt components contained in the still residue are completely removed therefrom.

As shown in the following examples, in accordance with the present invention, a high-purity di-TMP containing no coloring components is readily obtained. The thus obtained di-TMP is useful as a raw material of polyacrylate resins, polyether polyol resins, polyurethane resins, alkyd resins, synthetic lubricating oils or the like.

### SPECIFIC EXAMPLES OF THE INVENTION

The present invention will be described more specifically by reference to the following examples. However, these examples are only illustrative and not intended to limit the present invention thereto.

Meanwhile, in the following examples and comparative examples, the coloring degree was represented by a Hazen color scale and a Gardner color scale measured according to JIS K-0071-1 and JIS K-0071-2, respectively, and the "%" and "ppm" were values on a weight basis.

### PRODUCTION EXAMPLE

TMP was produced by reacting n-butyraldehyde with formaldehyde by the method described in Japanese Patent Application Laid-open No. Heisei 11 (1999)-49708. After completion of the reaction, the raw materials as low-boiling components were recovered and by-products were removed from the reaction solution, and then the resultant crude TMP was distilled by a film evaporator. The obtained still residue had the following composition:

| | |
|---|---|
| TMP | 10.0% |
| di-TMP | 39.6% |
| bis-TMP | 30.8% |
| Other organic by-products | 19.6% |
| Salts | 3,000 ppm |
| Coloring degree (Gardner color scale) | 18 |

### EXAMPLE 1

A 50-ml Erlemmeyer flask was charged with 10.0 g of the still residue obtained in PRODUCTION EXAMPLE, 3.0 g of hydroxylamine sulfate and 17.0 g of water, and the contents of the flask were heated at 90°C for 3 hours under stirring to conduct an acid decomposition reaction thereof. After completion of the reaction, the reaction mixture was measured by gas chromatography (hereinafter referred to merely as "GC") to examine its composition. As a result, it was confirmed that no di-TMP was decomposed, but bis-TMP and other components similar to di-TMP were completely decomposed and converted into low-boiling components such as TMP. In addition, it was confirmed that no CDF was produced by the acid decomposition reaction. After neutralizing the reaction mixture with a saturated sodium hydrogencarbonate aqueous solution, low-boiling and high-boiling components were removed therefrom, so that di-TMP having a purity of 98.3% and a Hazen color scale of 50 or lower was obtained at a recovery percentage of 80%.

### EXAMPLE 2

A 1,000-ml three-neck flask equipped with a reflux condenser and a magnetic stirrer, was charged with 201.3 g of the still residue obtained in PRODUCTION EXAMPLE, 404.9 g of methanol and 4.0 g of sulfuric acid. The contents of the flask were heated under stirring at such a temperature capable of causing methanol to be refluxed, thereby subjecting them to acid decomposition reaction for 2 hours. Dimethoxymethane produced during the reaction was continuously discharged out of the reaction system. After completion of the reaction, the obtained reaction mixture was measured by gas chromatography (GC) to examine its composition. As a result, it was confirmed that bis-TMP and other components similar to di-TMP were completely decomposed, and that the ratio of di-TMP to CDF was 4.3:1. After the reaction mixture was neutralized with a saturated sodium hydrogencarbonate aqueous solution, low-boiling and high-boiling components were removed therefrom. As a result, di-TMP having a purity of 98. 5 %and a Hazen color scale of 50 or lower was obtained at a recovery percentage of 60%.

### COMPARATIVE EXAMPLE 1

A 500-ml beaker equipped with a mechanical stirrer was charged with 150 g of the still residue obtained in PRODUCTION EXAMPLE and 150 g of methylisobutyl ketone (MIBK), and the contents of the flask were heated under stirring until a transparent solution was obtained. Then, the resultant solution was slowly cooled to 20°C for about 3 hours under stirring. The obtained crystals were washed with 75 g of MIBK and then dried under reduced pressure to recover about 90% of di-TMP and bis-TMP contained in the still residue. Sixty six grams of the obtained crystals were dissolved in 132 g of water at 50°C, and the resultant solution was slowly cooled to 20°C under stirring. The obtained crystals were washed with 66 g of water and then dried. As a result, it was confirmed that 30.9 g of crystals having a purity of 90.1% or higher when measured by GC and a Hazen color scale of about 130 were obtained, and the recovery percentage of di-TMP was 40%.

Meanwhile, EXAMPLE 1 shows the case where the acid decomposition was conducted by adding a scavenger for decomposed products to the still residue produced upon the purification of TMP; EXAMPLE 2 shows the case where the acid decomposition was conducted without using the scavenger for decomposed products; and COMPARATIVE EXAMPLE 1 shows the case where no acid decomposition was conducted. From the above results, it was confirmed that when the scavenger for decomposed products was added to the still residue upon the acid decomposition thereof in accordance with the present invention, it became possible to effectively remove impurities therefrom and obtain a high-purity di-TMP at a high yield.

### EXAMPLE 3

To 99.5 g of the still residue obtained in PRODUCTION EXAMPLE were added 99.9 g of water and 1.0 g of hydroxylamine sulfate. The mixture was heated to 90°C to dissolve the salt in water. The resultant solution was subjected to acid decomposition reaction for 6 hours while keeping the temperature thereof at 90°C. The reaction mixture was neutralized with sodium hydrogencarbonate, dehydrated and heated to 120°C under a pressure of 13 Pa or lower to remove low-boiling components therefrom. The obtained solution was subjected to film evaporator at 220°C under a pressure of 13 Pa or lower to remove high-boiling components and salts therefrom. Two parts by weight of ethyl acetate was added to one part by weight of the resultant distillate, and the obtained mixture was heated under stirring until a transparent solution was obtained. The obtained solution was gradually cooled under stirring for 6 hours to recover crystals. The resultant crystals were filtered out, washed with ethyl acetate and then dried to obtain 14.7 g of di-TMP. The measurement of the thus obtained di-TMP by gas chromatography showed that the purity thereof was 98.5% and the crystals exhibited a Hazen color scale of 50 or lower. The recovery percentage of di-TMP was 55%

### COMPARATIVE EXAMPLE 2

Five hundreds grams of the still residue obtained in PRODUCTION EXAMPLE was dissolved in 1,000 g of ethyl acetate under heating. The resultant solution was slowly cooled to 20°C while stirring. The obtained crystals were filtered out, washed with ethyl acetate and then dried. The resultant crystals had a brown color and contained 51.8% of di-TMP and 40% of bis-TMP. In addition, the crystals exhibited a Hazen color scale of 120 and the recovery percentage of di-TMP was 85%

EXAMPLE 3 shows the case where the still residue obtained upon the purification of TMP was sequentially subjected to acid decomposition, removal of high-boiling components from di-TMP and crystallization using ethyl acetate. It was confirmed that di-TMP obtained in EXAMPLE 3 had a higher purity as compared to that obtained in COMPARATIVE EXAMPLE 2 where the still residue was subjected to only crystallization using ethyl acetate.

### EXAMPLE 4

The still residue obtained in PRODCUTION EXAMPLE was subjected to film evaporator at 235°C under a pressure of 13 Pa or lower to distil off di-TMP and remove high-boiling components and salts therefrom. To 49.0 g of the thus obtained distillate was added 0.3 g (about 0.6%) of p-toluenesulfonic acid (PTSA), and the resultant mixture was subjected to acid decomposition at 90°C for 6 hours. The obtained reaction mixture was mixed with 98.0 g of ethyl acetate, and was stirred under heating until a transparent solution was obtained. The composition of the reaction mixture obtained after the acid decomposition reaction was as follows:

| | |
|---|---|
| TMP | 31.7% |
| di-TMP | 40.3% |
| CMF | 14.6% |
| bis-TMP | 0.0% |
| Other organic by-products | 13.4% |

Then, the reaction mixture was slowly cooled to room temperature (20°C) for 4 hours while stirring. The resultant crystals were filtered out, washed with 49.0 g of ethyl acetate two times, and then dried to obtain 5.9 g of crystals. The thus obtained crystals were di-TMP having a purity of 96.2%, and exhibited a Hazen color scale of about 20. The recovery percentage of di-TMP was 40%

### EXAMPLE 5

A 500-ml beaker equipped with a magnetic stirrer was charged with 90.0 g of the still residue obtained in PRODUCTION EXAMPLE and 90.0 g of ethyl acetate, and the contents of the beaker were heated to a temperature of 50 to 60°C while stirring to completely dissolve the still residue in ethyl acetate. The obtained solution was gradually cooled to 20°C for 6 hours while stirring to obtain crystals. The resultant crystals were filtered out, and washed with ethyl acetate. The thus obtained crystals were heated to 100°C to remove residual ethyl acetate. After removing the solvent, 270 mg (5,000 ppm) of PTSA was added to the crystals, and the resultant mixture was subjected to acid decomposition reaction at 90°C for 6 hours. The composition of the reaction mixture obtained after the acid decomposition reaction was as follows:

| | |
|---|---|
| TMP | 32.6% |
| di-TMP | 46.1% |
| CMF | 10.7% |
| bis-TMP | 0.0% |
| Other organic by-products | 10.6% |

The reaction mixture obtained after the acid decomposition was mixed with 60.0 g of ethyl acetate, and then heated to a temperature of 50 to 60°C while stirring to obtain a uniform solution. The solution was then gradually cooled to 20°C while stirring for 4 hours to obtain crystals. The obtained crystals were filtered out, washed with ethyl acetate, and then dried under reduced pressure to obtain 15.2 g of white di-TMP powder having a purity of 98.5%. Also, it was confirmed that the obtained di-TMP was a high-purity product exhibiting a melting point of 109.2 to 110.2°C and exhibited a Hazen color scale 20 or lower. The recovery percentage of di-TMP was 42%

### EXAMPLE 6

A 500-ml beaker equipped with a magnetic stirrer was charged with 180 g of the still residue obtained in PRODUCTION EXAMPLE and 180 g of methylisobutyl ketone (MIBK), and the contents of the beaker were heated to a temperature of 50 to 60°C while stirring to completely dissolve the still residue in MIBK. The resultant solution was gradually cooled to 20°C for 4 hours while stirring to obtain crystals. The obtained crystals were filtered out, washed with MIBK and then dried to obtain 94 g of crystals. After heating the crystals to 100°C, 330 mg (3,500 ppm) of p-toluenesulfonic acid was added thereto, and the mixture was subjected to acid decomposition reaction at 90°C for 2 hours.

The reaction solution obtained after the acid decomposition reaction was distilled at 140°C under a pressure of 13 Pa to recover low-boiling components (mainly CMF) therefrom. Then, the solution was cooled to about 100°C, and 110 g of MIBK was added thereto. The resultant mixture was stirred under heating at about 100°C to obtain a uniform solution. The obtained solution was cooled to 20°C for 3 hours while stirring to obtain crystals. The crystals were filtered out, washed with MIBK, and then dried under reduced pressure to obtain 45.2 g of white di-TMP powder having a purity of 98.4% and a Hazen color scale of 30 or lower. The recovery percentage of di-TMP was 63%

### EXAMPLE 7

The still residue obtained in PRODUCTION EXAMPLE was subjected to film evaporator at 235°C under a pressure of 13 Pa or lower to remove high-boiling components having a higher boiling point than that of di-TMP together with salts therefrom. The obtained distillate was further distilled at 180°C under a pressure of 13 Pa or lower to recover TMP therefrom. Forty nine grams of water was added to 49.0 g of the resultant bottoms, and the mixture was heated while stirring until a transparent solution was obtained. The obtained solution was slowly cooled to 0°C for 6 hours while stirring. The resultant crystals were filtered out, washed with water, and then dried to obtain 5.9 g of crystals. It was confirmed that the crystals were di-TMP having a purity of 95.4%, and exhibited a Hazen color scale of 50 or lower. The recovery percentage of di-TMP was 26%

### COMPARATIVE EXAMPLE 3

One thousand grams of the still residue obtained in PRODUCTION EXAMPLE was dissolved in 1,000 g of ethyl acetate under heating. The obtained solution was slowly cooled to 20°C while stirring, and further continuously stirred at 20°C for a while. After 6 hours, the obtained crystals were filtered out, sufficiently washed with 1,000 g of ethyl acetate, and then dried to obtain 52 g of crystals. It was confirmed that the resultant crystals contained 57.1% of di-TMP and 39.3% of bis-TMP, and exhibited a Hazen color scale of about 150. The recovery percentage of di-TMP was 75%.

EXAMPLE 4 shows the case where the still residue obtained upon the purification of TMP was successively subjected to film evaporator in order to remove high-boiling components therefrom, to acid decomposition in order to decompose a formal compound contained therein, and then to crystallization; EXAMPLES 5 and 6 shows the case where the still residue obtained upon the purification of TMP was successively subjected to crystallization using a solvent, to acid decomposition and then again to crystallization; and EXAMPLE 7 show the case where the still residue obtained upon the purification of TMP was subjected to film evaporator in order to remove high-boiling components therefrom, and then to crystallization. From the above results, it was confirmed that di-TMP obtained in each of these EXAMPLES exhibited a lower coloring degree and had a higher purity as compared to that obtained in COMPARATIVE EXAMPLE 3 where the still residue was subjected only to crystallization.

## Claims

1. A process for recovering ditrimethylolpropane from a still residue obtained by extracting and then distilling off trimethylolpropane from a reaction solution obtained by reacting n-butyraldehyde with formaldehyde in the presence of a basic catalyst, said process comprising:
subjecting a formal compound contained in the still residue to acid decomposition.

2. A process according to Claim 1, wherein the acid decomposition of the formal compound is performed at a temperature of 20 to 180°C using a mineral acid and/or an organic acid.

3. A process according to Claim 2, wherein at least one compound selected from the group consisting of alcohols and hydroxylamine salts is added to the still residue together with the acid.

4. A process according to any one of Claims 1 to 3, comprising the steps of:
i) subjecting the formal compound contained in the still residue to acid decomposition:
ii) removing high-boiling components having a higher boiling point than that of ditrimethylolpropane, from the still residue; and
iii) subjecting the resultant product to crystallization using a solvent.

5. A process according to any one of Claims 1 to 3, wherein after the formal compound contained in a distillate obtained by removing high-boiling components having a higher boiling point than that of ditrimethylolpropane from the still residue, is subjected to acid decomposition, the resultant product is subjected to crystallization using a solvent.

6. A process according to Claim 4 or Claim 5, wherein the removal of the high-boiling components having a higher boiling point than that of ditrimethylolpropane, is performed by molecular distillation.

7. A process according to any one of Claims 1 to 3, wherein after the still residue is first subjected to crystallization using a solvent and then the formal compound contained in the resultant crystallized product is subjected to acid decomposition; the resultant reaction mixture obtained from the acid decomposition is subjected to crystallization.

8. A process for recovering ditrimethylolpropane from a still residue obtained by extracting and then distilling off trimethylolpropane from a reaction solution obtained by reacting n-butyraldehyde with formaldehyde in the presence of a basic catalyst, said process comprising:
removing high-boiling components having a higher boiling point than that of ditrimethylolpropane, from the still residue; and
subjecting the resultant product to crystallization.

9. A process according to Claim 8, wherein the removal of the high-boiling components having a higher boiling point than that of ditrimethylolpropane, is performed by molecular distillation.

10. A process according to Claim 8 or Claim 9, wherein the solvent used upon the crystallization is water.
